Europäisches Patentamt

European Patent Office

Office européen des brevets

(11) Publication number: **0 347 230**
**A2**

# EUROPEAN PATENT APPLICATION

(21) Application number: 89306084.8

(22) Date of filing: 15.06.89

(51) Int. Cl.⁴: **C 12 N 15/00**
**C 12 P 21/00, G 01 N 33/577**

(30) Priority: 17.06.88 US 208243

(43) Date of publication of application:
20.12.89 Bulletin 89/51

(84) Designated Contracting States:
AT BE CH DE ES FR GB GR IT LI LU NL SE

(71) Applicant: BIOGENEX LABORATORIES
4600 Norris Canyon Road
San Ramon California 94583 (US)

(72) Inventor: Kalra, Satya
4515 Kingswood Drive
Danville California 94526 (US)

Key, Marc
155 Cabro Court
Novato California 94947 (US)

Kalra, Krishan L.
4515 Kingswood Drive
Danville California 94526 (US)

(74) Representative: Harrison, David Christopher et al
MEWBURN ELLIS 2 Cursitor Street
London EC4A 1BQ (GB)

(54) Miniclones.

(57) A method of preparing antibody-producing cells of transformed B-lymphocytes for use in diagnosis or therapy, along with the antibodies produced thereby, is disclosed. The method comprises creating a collection of antibody-producing cells from B-lymphocytes sensitized with the antigen, separating the collection of cells into populations comprising a plurality of antibody-producing cells without passing through a monoclonal stage, and selecting from the populations a miniclone that produces a plurality of antibodies of different specificities reactive with the antigen.

**Description**

## MINICLONES

This invention relates to the production of antibodies.

A number of diagnostic assays rely on the ability of reagents to react in a specific manner to identify a particular molecule or an organism associated with a particular molecule. The mammalian immune system has a matchless ability to produce, for use as such reagents, molecules with specificity and avidity for other molecules that have a particular spatial and polar structure, as may be found with sequences of amino acids and sugars. For a long period of time, one was dependent upon producing antisera containing polyclonal antibodies by employing the immune system in vivo. The animal producing the antiserum can be selected to provide polyclonal antibodies demonstrating high specificity for a specific antigen, but antisera from such animals usually cannot discriminate between various sites on the antigen, as it comprises a mixture of antibodies of varying specificity and avidity. Thus, one observed the average antibody-antigen interaction over the entire composition and not the properties of a specific antibody. Antibody production as an antiserum is further limited by the amount that can be produced by the immunized animal. Furthermore, the properties of antisera vary from animal to animal, which causes problems in producing standard assays that depend on specific antibodies.

With the seminal discovery by Milstein and Kohler, one can now produce homogeneous compositions of antibodies by fusing a B-lymphocyte with a myeloma cell to produce a cell referred to as a hybridoma. The initially produced cells are subjected to limiting dilution or other techniques that ensure that all cells in a given collection arise from a single parent cell. The resulting antibody-producing cells are thus referred to as monoclonals. Monoclonal antibodies have constant specificity and avidity and are particularly useful for diagnostic assays that require high specificity for distinguishing between similar antigens or similar epitopes on a single antigen. On the other hand, reactivity of monoclonal antibodies with a single antigenic determinant has caused problems in developing immunoassays for routine clinical application, particularly assays for polymorphic materials (such as influenza virus or other viruses that have multiple forms where it is desirable that the antibody should bind to multiple antigenic forms).

In some instances, mixtures of monoclonal antibodies have been made in order to increase the range of binding specificities. However, such a technique is generally unsatisfactory as it significantly increases the number and amount of laboratory manipulation that must occur before a final working composition is obtained. Accordingly, new techniques that provide antibodies having high affinity and broad specificity while avoiding the variability that exists in traditional polyclonal antibodies remain a desirable object despite the work of numerous investigators in this field.

## SUMMARY OF THE INVENTION

Accordingly, the present invention provides a method for preparing antibody for use in diagnosis or therapy involving polymorphic or multiple-determinant antigen, which comprises creating a collection of immortalized antibody-producing cells from B-lymphocytes sensitized with said antigen; separating said collection into miniclones comprising a plurality of antibody-producing cells without passing through a monoclonal stage; and selecting from said miniclones a miniclone that produces a plurality of antibodies of different specificities reactive with said antigen. The selected miniclone can be expanded readily to provide a corresponding mixture of antibodies, referred to as miniclonal antibodies, having the desired high specificity but being sufficiently heterogeneous to provide for diagnosis or therapy involving polymorphic or multiple-determinant antigen. Multiple miniclones reactive with the antigen can be combined and expanded, if so desired.

## DESCRIPTION OF SPECIFIC EMBODIMENTS

One factor leading to the present invention was the realization by the inventor that limiting dilution and similar techniques designed to isolate a single clone from a collection of antibody-producing cells are not required in order to produce desirable antibodies. The present invention accordingly represents the elimination of a step previously considered to be essential for the production of useful antibodies by techniques involving the production of antibody-secreting clones.

In a typical process for the production of monoclonal antibodies of the Kohler and Milstein variety, B-lymphocytes are sensitized with the antigen of interest in order that antibody of the desired specificity will be produced. Specific binding, as used herein, refers to binding that takes place at the specific binding site of an antibody, as opposed to non-specific binding that can occur at other locations on the antibody molecule. The B-lymphocytes can be either peripheral lymphocytes, spleen cells, or other cells obtained in vivo or can be lymphocytes used in an in vitro sensitization technique. The use of antigens to sensitize B-lymphocytes is well known in the art and does not represent part of the present invention.

The B-lymphocytes may be fused with a myeloma cell to produce an immortalized hybridoma cell with infinite capacity for in vitro replication and antibody production. Alternatively, B-lymphocytes can be immortalized by an appropriate transforming vector such as Epstein-Barr virus. The immortalization of B-lymphocytes by fusion with myeloma cells or by viral transformation to produce a collection of antibody-producing cells is likewise old and in the prior art.

In the prior art technique for production of monoclonal antibodies using hybridomas, a typical fusion procedure will result in the production of three different types of hybridomas: 1) non-antibody producers, 2) non-specific antibody producers, and 3) specific antibody producers. Of these three types of hybridomas, only the latter type is desirable, and much of the prior art teaching is directed to isolation of these specific producers from the two other types of cells. The first step in selecting an antibody-producing hybridoma cell typically was to carry out a separation of hybridoma cells from non-hybridoma cells, typically using a selective medium in which only hybridoma cells will grow continuously. Surviving cells were then divided into clumps of cells that were tested for production of desired antibody by binding with the antigen of interest.

However, in the present invention, those B-lymphocytes which are likely to give rise to non-antibody producers and non-specific antibody producers are reduced or eliminated from the total B-lymphocyte population prior to fusion or immortalization. This separation is carried out by any technique that recognizes specific binding of antibodies that are present on the surface of the lymphocytes and can use that recognition to separate the specific lymphocytes from other cells, such as by specifically binding the B-lymphocytes to an antigen-coated solid absorbent (e.g., a plastic tissue culture dish). Those lymphocytes which display specific antigen recognition by binding to the antigen-coated absorbent possess the desired qualities (e.g., specificity and antibody secretion) for antibody production and are then collected for use in preparing immortalized B-lymphocytes.

In the present technique, the lymphocytes can be immortalized by fusion with myeloma or lymphoblastoid cells (hybridoma technique) or by a transforming vector such as a virus. These immortalized cells are then subdivided into multiple separate populations. The subdivision is planned so that each isolated population is of restricted clonal composition but is not monoclonal, such that each isolated population has the potential capacity to produce from one to several different types of antibody molecules. By employing appropriate testing techniques (discussed below), those individual populations capable of producing the desired antibodies are identified for use and/or are pooled together to form a combined population of antibody-producing cells, whereas those populations which do not produce the desired types of antibodies are discarded.

Within the combined population so formed by pooling individual populations (or uncombined individual populations already containing multiple antibody-type producers) are a plurality of cells producing desired antibodies. However, some cells may exist within the combined population which produce no antibody or non-specific antibody. These undesired cells can be reduced or eliminated by selecting and saving only those cells which are capable of binding to antigen-coated solid surfaces.

The saved cells are referred to herein as refined miniclones to indicate that this additional selection technique has been used. By employing this antigen binding procedure for the selection of desired lymphocyte populations, it is possible to select predominantly those clones of cells which produce the specific antibodies of interest while eliminating the majority of undesirable clones. Furthermore, it is possible to select said desired clones without going through a cloning procedure and the production of monoclones.

These groupings of cells, referred to as compound clones or miniclones to distinguish them from monoclonal cells, are specifically selected by any technique that will ensure the presence of multiple antibodies that react with the antigen of interest. The miniclonal antibodies so produced will react with antigen, whether that antigen is monomorphic, polymorphic, or comprises multiple determinants. For example, if a polymorphic virus is the antigen of interest, the ability of the supernatant (which contains antibodies) from a particular miniclone to bind with multiple strains of virus can be tested. Each of the individual assays will be a traditional assay of the type used for selecting clones in monoclonal antibodies. However, a miniclone by virtue of the selection procedure will have a plurality of antibodies of different specificities reactive with the antigen of interest.

Although production of monoclonal antibodies also involves the initial recognition of collections of antibodies that react with particular antigen, specific selection of a plurality of cells for expansion into antibody-producing cells is a step that is deliberately avoided in the production of monoclonal antibodies. Instead, limiting dilution or similar techniques are deliberately used to ensure production of cell lines rising from single cells in order to avoid problems associated with non-producers and non-specific producers of antibody that might also be present. Even when monoclonal antibodies have been produced for later production of a mixture of monoclonal antibodies, all such selections have passed through a monoclonal stage in contrast to the technique of the present invention.

The miniclonal antibodies of the present invention can be obtained from any convenient vertebrate source (host), such as murine, primate, e.g., human, lagomorph, bovine, ovine, equine, porcine, etc. The antibodies can be prepared by fusing lymphocytes from a host having elevated serum levels of antigen-specific antibodies with myeloma cells in accordance with known techniques or by transforming the lymphocytes of such a host with an appropriate transforming vector to immortalize the cells. The cells may be cultured in a selective medium and screened to select antibodies that bind the desired antigen.

The fusion partner may be any convenient immortalized B-cell which preferably does not secrete whole immunoglobulin molecules and which can be selected against, as with HAT medium, and desirably which has a high fusion efficiency. Illustrative fusion partners are UC729-6, J-4 (SKO-007), GM1500 6TG-A12, P3/NS1/1-Ag4-1, and SP2O-Ag14.

The fusions may be performed as described in the literature employing polyethylene glycol as fusogen, plating the cells in HAT medium in a plurality of wells, and then screening supernatants in the viable cell-containing wells for antibodies of interest. Wells positive for reactivity are then expanded without limiting dilution or other techniques designed to produce monoclonality. The resulting hybridomas are referred to as compound hybridomas to distinguish from monoclonal hybridomas. The mixture of antibodies produced by compound hybridomas is referred to as miniclonal, to indicate that they are derived from a relatively small number of clones (abbreviated miAbs). Alternatively, lymphocytes may be immortalized with a transforming vector such as Epstein-Barr virus or by any combination of viral transformation and cell fusion technique.

The selection technique used to obtain antibody-producing cells at the various stages discussed above can be any technique that will allow separation of antibody-producing cells of a desired specificity from other cells. Various techniques exist in the prior art, such as binding of antibody-producing cells to antigens attached to solid surfaces, cell sorting based on ability to bind to fluorescent-labeled antibodies, and the like. Use of antigens bound to solid surfaces represents a preferred embodiment because of the simplicity of the technique. Antigens of interest are attached to a solid surface (numerous techniques exist, some of which are described in patents and other publications referenced later in the description) and a suspension of cells to be tested is contacted with the surface. Non-binding cells are removed by gentle washing, after which bound cells are removed separately. The exact technique will vary according to the type of surface used. For example, beads or similar materials may be used in a chromatography process or continuous solid surfaces, such as container walls, can be used with fluid removal taking place by aspiration or decanting.

Selected miniclones are expanded, i.e., grown into larger numbers of cells for use in antibody production, according to standard techniques well described in scientific and patent literature.

The miniclonal antibodies of this invention can find use in a variety of ways, particularly as sources of reagents and as precursors to products which find use as reagents.

The miniclonal antibodies can be used in both in vivo and in vitro diagnosis, as well as in therapy. For many applications, the antibodies will be labeled with a compound which imparts a desired property to the antibodies, such as providing a detectable signal, providing cytotoxicity, providing for localized electromagnetic radiation, or the like.

A wide variety of labels are known which provide for a detectable signal. Illustrative labels include radioisotopes, e.g., 3H, 125I, 131I, 14C; fluorescers, e.g., fluorescein, phycobiliproteins, rare earth chelates, rhodamine; enzyme substrates and inhibitors; enzymes, such as horseradish peroxidase, glucose oxidase, glucose-6-phosphate dehydrogenase, β-galactosidase; acetylcholinesterase; particles, e.g., dextran, agrose, metal particles, carbon magnetic particles, polystyrene particles; or the like. Methods for conjugating labels to proteins have found extensive description in the literature; see, for example, U.S. Patent Nos. 3,817,837; 4,134,792; and 4,220,722.

The labeling employed will follow conventional techniques, and the number of labels per antibody molecule will vary depending upon the nature of the label, the sensitivity of the signal desired, the purpose of the labeling, and the like. Numerous assay protocols have been developed for use with antibodies for the detection of a wide variety of analytes which are applicable here. See, for example. U.S. Patent Nos. 3,791,932; 3,817,837; 4,134,792; 4,174,384; 4,275,149; and 4,299,916, which are incorporated herein by reference.

The miniclonal antibodies may be incorporated in liposome membranes or modified with lipids, so as to be incorporated in such membranes. The antibodies by themselves or labeled may be used in in vitro diagnosis for measuring the presence of antigens associated with, for example, a neoplasm such as cervical cancer, for in vivo diagnosis for introduction into a host, e.g., intravenously in a physiologically acceptable carrier, e.g., phosphate buffered saline (PBS), or may be introduced for therapeutic purposes in the same manner.

In addition to use in assays, miniclonal antibodies can be used for any other technique for which monoclonal and/or polyclonal antibodies are currently being used, including use as specific binding agents for in vivo therapeutic use (typically with a radioactive or radioopaque label or with a cytotoxic label intended for destruction of target cell).

The antibodies, by themselves or labeled, can also be used for treating a neoplasm in a human or animal, particularly mammalian, host such as cervical carcinoma, prostate tumor, colon carcinoma, lung cancer, breast cancer or melanoma. See, for example, U.S. Patent Nos. 4,331,647; 4,348,376; 4,361,544; 4,468,457; 4,444,744; 4,460,559; and 4,460,561, all of which describe a number of techniques for diagnosis and treatment of tumors using radioactively labeled antibodies. Other uses specifically include diagnosis and treatment of viruses in viral diseases, particularly diseases caused by polymorphic viruses, such as influenza virus or HIV-III (the virus believed to be responsible for acquired immunodeficiency syndrome; AIDS).

The antibodies of this invention are easily soluble in physiological saline, and therefore can be injected intravenously or intramuscularly as a saline solution or a drip. Furthermore, the antibodies of the invention can be used in the form of an ointment or suppository.

The amount and the binding affinity (or avidity) of antibody employed will vary depending upon the particular application. Use of antibodies for diagnostic and therapeutic purposes has been extensively described in the literature.

The entire antibody need not be used; for many applications only a fragment having intact variable regions will suffice. For example, Fab fragments, F(ab′)2 fragments, or Fv fragments may suffice. Additionally, chimeric antibodies having the variable regions of the intact antibodies can be produced.

The antibodies of the invention can be used in a number of specific qualitative and quantitative immunochemical methods such as immunoelectrophoresis, immunodiffusion, immunoprecipitation, complement fixation, hemagglutination, radioimmunoassay, enzyme-linked immunosorbant assay, and the like. These assays have been developed over the past several years for the determination of the presence and/or amount of various antigens. More recently developed techniques for the study of antigen-antibody reactions include immunoradiometric assays as well as other types of immunometric assays.

One advantageous immunometric assay is the two-site or "sandwich" immunometric assay in which an antigen is reacted with an antibody that is immobilized on a solid surface. A second antibody to the antigen is labeled and is reacted with the antigen which is bound to the solid support through the first antibody.

This technique was originally developed using polyclonal antibodies produced in traditional in vivo techniques. More recently, monoclonal antibodies have been applied to sandwich assays. However, the aforementioned disadvantages of monoclonal antibodies are seen in such assays.

By applying miniclonal antibodies to sandwich assays, many of the disadvantages of both the polyclonal and the monoclonal systems can be avoided.

The miniclonal antibodies of the invention can be partially or completely purified by known methods, such as precipitation with polyethylene glycol or a saturated solution of ammonium sulfate, followed if desired by dialysis or affinity chromatography.

The present invention comprises both the compound clones (miniclones) described herein that have been selected as described as well as the miniclonal antibodies produced thereby. Techniques involving miniclonal antibodies are also part of the present invention. One aspect of the present invention comprises a test or an in vitro diagnostic kit containing various components and reagents such as miAbs in liquid- or solid-phase storage forms, either labeled or unlabeled; various antigens for use as positive or negative standards; buffers, such as phosphate, citrate, Tris, HEPES, and the like; and other additives or reagents formerly required to perform the particular assay in which the miAbs are used. The kit components may optionally include stabilizers, such as bovine serum albumin, human serum albumin, dextrose, sodium azide, or the like, as well as other reagents typically found in immunoassay kits.

The invention now being generally described, the same will be better understood by reference to the following detailed examples which are provided for the purposes of illustration only and are not to be considered limiting of the scope of the invention unless so specified.

### EXAMPLES

### Example 1

### Production of Miniclonal Antibodies Specific for Influenza Virus

Miniclonal anti-influenza virus (anti-flu) antibodies can be obtained by fusing spleen cells from MRL/lpr mice with the FOX-NY myeloma cell line as previously described (Taggart and Samloss, Science (1983) 219:1228-1230; Weisbart, et al., J. Immunol. (1984) 132:2909-2912). Briefly, spleen cells ($2.4 \times 10^8$) are obtained from MRL/lpr mice (Jackson Laboratories, Bar Harbor, Maine) with elevated serum levels of anti-flu antibodies. Spleen cells are allowed to bind to antigen-coated Petri dishes, and nonadherent cells are discarded. Antigen-binding cells are recovered from the plates and fused with $4.8 \times 10^7$ myeloma cells using 50% polyethylene glycol 1600. The cells are cultured at $1 \times 10^6$/ml in 96-well plates in 0.2 ml RPMI 1640 and 10%-fetal calf serum. Culture supernatants are harvested after 10 days and assayed for IgG antibodies to influenza virus by enzyme-linked immunosorbant assay (ELISA) as previously described (Weisbart, et al., Annals Int. Medicine (1986) 104:310-313). Screening is designed to select only IgG antibodies that bind virus. Broad binding specificity is ensured by selecting against several viral strains. By combining two or more wells that produce antibody of desired specificity, the multiclonal nature of the combined population is ensured. The combined population of cells is then allowed to bind to antigen-coated Petri dishes, and all unbound cells are discarded. Antigen binding cells are then collected and expanded into miniclones producing the desired antibodies. Miniclonal antibodies are assayed at dilutions (0.5-10 µg/ml) that give comparable responses by ELISA. Minicloned cells (about $10^7$) producing the selected miAbs are injected into the peritoneal cavities of pristane-primed Balb/c mice, and the miAbs are purified from ascites by binding to protein-A sepharose and removing non-immunoglobulin proteins by washing with 0.5 M NaCl in phosphate buffer, pH 7.2, followed by 0.1 M Tris-glycine HCl, pH 4.0. For example, a miAb may be eluted from protein-A with 0.1 M Tris-glycine HCl, pH 2.8.

### Example 2

### Fusion and Selection of Hybridoma Miniclones Secreting Antibodies Specific for Carcinoma Cells

Lymph nodes from cancerous patients are teased with nugent forceps in RPMI 1640 media, and isolated lymphocytes are cultured overnight at 37°C and 5% $CO_2$ in RPMI 1640 with 10% fetal calf serum (FCS) and 2 mM L-glutamine. On the bottom of the culture vessel is a monolayer of cancer cells (antigen) used for binding antigen-reactive lymphocytes. After overnight incubation, unbound lymphocytes are discarded, and antigen-binding lymphocytes are collected. Lymphocytes are counted and mixed at a ratio of 2:1 with the human lymphoblastoid B cell line UC729-6 (Handley and Royston, 1982, in "Hybridomas in Cancer Diagnosis and Treatment," eds. Mitchel and Oettgen, pp. 125-132, Raven Press, NY), then fused with polyethylene glycol 1500 by a modification of the technique by Gefter et al., Somatic Cell Genetics (1977) 3:321-336, as described below). Fused cells are plated at 105 cells/well in a Costar 96-well microtiter plate with Hypoxanthine-Amethopterin-Thymidine (HAT, Littlefield, Science (1964) 145:709-710) supplemented RPMI 1640 with 10% FCS and L-glutamine. Within 10-20 days, wells positive for hybridoma growth are assayed for human antibody production (using an anti-human antibody), and their reactivity to a human cell panel is determined by an enzyme immunoassay (EIA). Hybridomas producing the desired antibody are pooled together and then selected for antigen-binding on antigen-coated Petri dishes. Unbound cells are discarded and bound cells are collected into miniclones and expanded for further study without limiting dilution or otherwise passing through a monoclonal stage.

Enzyme Immunoassay

Human miAbs and their reactivity to cells are detected by a modification by an EIA previously described (Handley et al., J. Immunologic Methods (1982) 54:291-296, as modified by Glassy et al., J. Immunologic Methods (1983) 58:119-126). Briefly, 50 $\mu$l of either an affinity-purified goat anti-human Ig or a $4 \times 10^6$ target cell/ml suspension is immobilized in triplicate wells of an immunofiltration manifold. (The specially designed microtiter plate which serves as both an incubation chamber and filtration manifold (VP No. 107) is available commercially from V and P Sci entific, San Diego, CA). The bottom of each well contains a 0.6 mm hole over which is placed a 6 mm diameter glass fiber filter. Surface tension prevents fluid volumes less than 100 $\mu$l from draining through the hole until a vacuum is applied. When vacuum is applied, fluid is drawn through the filter and out the drain hole leaving particulate matter trapped on the filter. After washing 3 times with 0.3% gelatin in phosphate buffered saline, 50 $\mu$l of hybridoma supernatant are incubated 30 minutes at room temperature. Filters are then washed and incubated with 50 $\mu$l of a horseradish peroxidase-conjugated goat anti-human Ig for an additional 30 minutes. Filters are washed again and incubated with 150 $\mu$l of a 400 $\mu$g/ml solution of ortho-phenylenediamine in citrate buffer. One hundred uL of each well are then transferred to a new plate containing 50 $\mu$l of 2.5 M $H_2SO_4$ and read on a Dynatek (Alexandria, VA) MR 580 micro-ELISA reader at 492 nm.

Hybridoma culture fluids are precipitated with 50% ammonium sulfate and crude Ig fractions collected. The precipitates are dissolved in physiological saline and purified by affinity chromatography using Staph. aureus Protein A-bound Sepharose with IgG and Sepharose-[sheep anti(human IgM) antibody] with Igm. Typically, at least 2 mg of miniclonal antibodies can be obtained from one liter of miniclonal culture fluid.

### Example 3

#### Preparation of Miniclonal Antibodies Against Human Chorionic Gonadotropin (hCG)

Miniclonal antibodies for hCG antigen are prepared by immunizing Balb/c mice at periodic intervals. Spleen cells (splenocytes) are prepared from the mice having the highest titers of anti-hCG antibodies in sera. Prior to fusion, the spleen cells are selected for binding to antigen-coated Petri dishes, and unbound spleen cells are discarded. Those cells dis playing specific antigen binding are collected and fused with mouse myeloma cells (NS1) according to the procedures of Kohler and Milstein described above. Prior to fusion, myeloma cells are grown in a log phase in vitro in a culture medium consisting of Dulbecco's Modified Minimum Essential Medium (DMEM) with high glucose (Gibco #3201960AJ or equivalent), 3 mM sodium pyruvate, 5 mM 2-mercaptoethanol, 10 mM non-essential amino acids (Gibco #320-1140 or equivalent), 3 mM hypoxanthine, 0.3 mM thymidine, and 10% heat-inactivated fetal bovine serum (FBS; Gibco #200 6140 or equivalent). Gentamycin is used as an antibiotic. Cells are grown in a 5% $CO_2$ atmosphere at 37°C in tissue culture dishes or flasks. Cells grow most rapidly when maintained at densities between $0.5 \times 10^5$ cells/ml and $5.0 \times 10^5$ cells/ml. Cells are stored frozen in liquid nitrogen by suspending 1 to $10 \times 10^6$ cells in about 1 ml of a solution containing 10% dimethyl sulfoxide (DMSO), 20% heat-inactivated fetal calf serum, and 70% of DMEM medium with the additive specified above. The fusion produces a collection of hybrid myeloma cells (hybridomas) which are screened for the production of antibodies in vitro.

Screening for Specific Binding to hCG

The initial collection of hybridoma cells is divided to produce a series of small samples of multiple cells potentially containing a plurality of antibody-producing hybridoma cells (without passing through a monoclonal state). Supernatants from the individual samples (about 100 $\mu$l/sample) are added to a series of appropriately labeled tubes containing hCG standards, followed by the addition of about 100 $\mu$l of $^{125}$I-labeled polyclonal anti-hCG. For example, rabbit anti-hCG is available from commercial sources. The tubes are incubated at 37°C for about 30 minutes. The miAb-hCG-PAB complex is precipitated by 2 ml of goat anti-mouse antiserum containing 4% polyethylene glycol in a 0.1 M phosphate buffered solution. The precipitated miAb-hCG-PAB complex is then separated by centrifugation at 1500 x g for 10 minutes. The supernatant is decanted, and the reaction tubes containing the solid pellets are counted in a gamma scintillation counter.

This initial screening procedure determines whether antibodies specific for hCG are present. Cells from those wells containing the desired antibodies are pooled and subject to antigen selection by binding to antigen-coated Petri dishes. Only those cells displaying specific antigen binding are harvested and expanded for further study.

Miniclonal antibodies can be tested for multiple specificities to the hCG molecule by their ability to block the binding of more than one monoclonal antibody to hCG. This information is typically obtained by carrying out a blocking assay in which a suspected miniclone supernatant is allowed first to bind with hCG followed by addition of a labeled monoclonal antibody that is known to bind to hCG. If a particular antibody is present in the suspected miniclonal antibody that reacts with (or sufficiently near) the same epitope as the monoclonal antibody, the amount of monoclonal antibody that can bind to the hCG will be less than that which binds in the absence of the aliquot of suspected miniclonal antibody.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

The invention now being fully described, it will be apparent to one of ordinary skill in the art that many changes and modifications can be made thereto.

## Claims

1. A method of preparing antibody-producing hybridomas or transformed lymphoid cell lines for use in diagnosis or therapy involving a polymorphic or multiple-determinate antigen, which comprises:
creating a collection of antibody-producing cells from B-lymphocytes sensitized with said antigen;
separating from said collection of lymphocytes a population of lymphocytes that specifically bind said antigen;
transforming said population of lymphocytes that specifically bind said antigen by cell fusion or transforming vector into immortalized antibody-secreting cells;
separating said immortalized cells into multiple-clone populations;
identifying from said multiple-clone populations at least one miniclonal population that produces antibodies reactive with said antigen; and
expanding said miniclonal population without passing through a monoclonal stage.

2. The method of Claim 1, wherein more than one miniclonal population is identified and combined into a common population.

3. The method of Claim 2, wherein said common population is selected into a refined miniclone by binding to an antigen-coated solid surface and selecting cells capable of specific binding to the antigen of interest.

4. The method of Claim 1, wherein said antigen is a polymorphic virus.

5. The method of Claim 4, wherein said virus is an influenza virus or HIV.

6. The method of Claim 4, wherein said selecting comprises measuring the ability of said antibodies from said miniclone to bind specifically with at least two different polymorphic forms of said virus.

7. The method of Claim 1, wherein said antigen is a multiple-determinate antigen.

8. The method of Claim 6, wherein said selecting comprises measuring the ability of antibodies from said miniclone to block the binding on at least two monoclonal antibodies specific for different determinates on said antigen.

9. In a method of detecting in a sample the presence or quantity of a polymorphic or multiple-determinate antigen by an immunological reaction between said antigen and an immunological substance an improvement which comprises:
utilizing as said immunological substance a miniclonal antibody preparation, wherein said preparation comprises multiple antibodies having different specificities secreted by multiple clones of cells that have not passed through a monoclonal stage.

10. The method of Claim 9, wherein said immunological reaction is a sandwich assay.

11. A miniclonal antibody preparation, wherein said preparation comprises purified multiple antibodies having different specificities secreted by multiple clones of cells that have not passed through a monoclonal stage.